Europäisches Patentamt

European Patent Office

Office européen des brevets

Veröffentlichungsnummer: **0 275 072**
**A2**

# EUROPÄISCHE PATENTANMELDUNG

Anmeldenummer: **88100295.0**

Int. Cl.⁴: **G01N 1/10**

Anmeldetag: **12.01.88**

Priorität: **12.01.87 DE 3700654**
**19.02.87 DE 3705197**

Veröffentlichungstag der Anmeldung:
**20.07.88 Patentblatt 88/29**

Benannte Vertragsstaaten:
**AT CH DE IT LI**

Anmelder: **Jansky GmbH Zweigniederlassung
Emsdetten
Taubenstrasse 25-43
D-4407 Emsdetten(DE)**

Erfinder: **Jansky, Manfred, Dipl.-Ing.
Taubenstrasse 37
D-4407 Emsdetten(DE)**

Vertreter: **Patentanwaltsbüro Cohausz &
Florack
Postfach 14 01 47
D-4000 Düsseldorf 1(DE)**

Datenerfassungseinrichtung für von verschiedenen Lieferanten stammende, in einem Tank gesammelte Milch.

Die Erfindung bezieht sich auf eine Datenerfassungseinrichtung für Milch, die in Teilvolumina bei verschiedenen Lieferanten in einem Sammeltank eines Transportfahrzeugs gesammelt wird. Bei der Annahme werden von den Teilvolumina Proben in Probegefäße 8 abgefüllt, die in hintereinander, insbesondere im Kreis, oder nebeneinander angeordneten Stativelementen 9 hintereinander in Reihe fest positioniert sind. Mittels einer gemeinsam mit dem Abfüllelement 7 verfahrbaren Leseeinrichtung 17,24,25 sind sowohl eine Markierung 26 an einem jeden Stativelement 9 als auch längs der Reihen der Probegefäße 8 angeordnete Codes 18 auslesbar, so daß die Position eines jeden Probegefäßes 8 mit den Daten für die Probe eines jeden Probegefäße 8 von der Datenerfassungseinrichtung zusammengebracht werden kann, damit später im Labor die Daten den Probegefäßen 8 zugeordnet werden können.

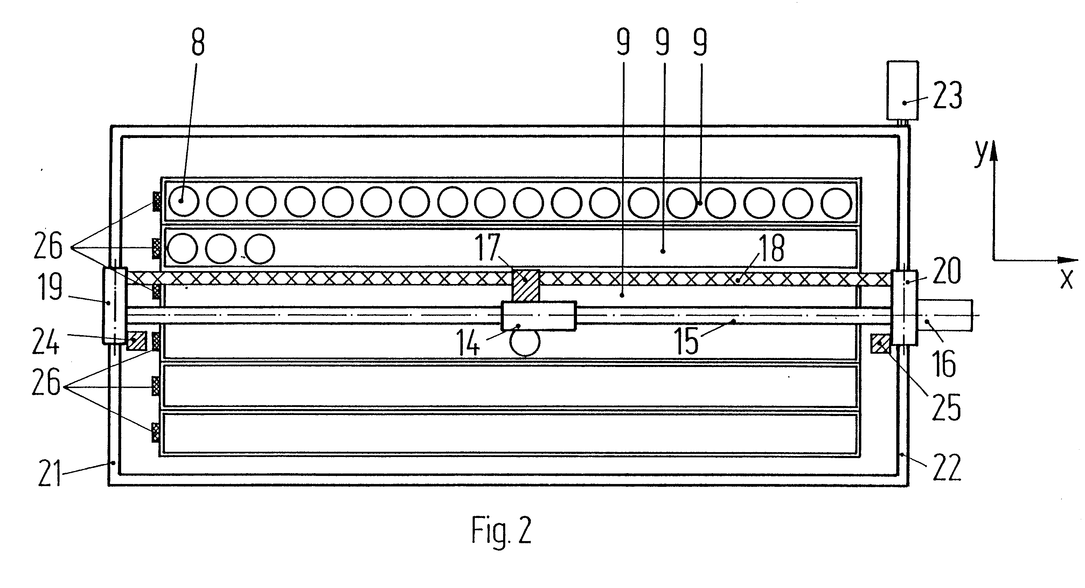

Für die Zusammenfassung ist Figur 2 bestimmt.

Fig 2

## Datenerfassungseinrichtung für von verschiedenen Lieferanten stammende, in einem Tank gesammelte Milch

Die Erfindung bezieht sich auf eine Datenerfassungseinrichtung für die Teilvolumina von bei verschiedenen Lieferanten in einem Sammeltank eines Transportfahrzeuges zu sammelnder Milch, mit einem Volumenmesser für die Teilvolumina, einer Probenahmevorrichtung zur Entnahme von Proben aus den Teilvolumina und einem Abfüllgerät für die Proben, das mit seinem Abfüllelement über den in einem Stativ fest positionierten Probegefäßen und relativ zu ihnen verfahrbar angeordnet ist, wobei die Datenerfassungseinrichtung die vom Volumenmesser für die Teilvolumina gelieferten Daten den von einem Positionsmelder gelieferten Daten für die Position des mit der entsprechenden Probe gefüllten Probegefäßes in ihrem Speicher zuordnet.

Bei der Annahme von Milch bei verschiedenen Lieferanten ist es notwendig, nicht nur das Milchvolumen festzustellen, sondern auch die Milchqualität. Für die Feststellung der Milchqualität wird von jedem angenommenen Milchvolumen eine insbesondere mengenproportionale Probe in ein Probegefäß abgefüllt. Es ist üblich, die Probegefäße in Stativen fest zu positionieren, wobei jedes Stativ eine Gefäßreihe aufnimmt. Mit dem in zwei Koordinaten verfahrenbaren Abfüllelement kann jedes Probegefäß angefahren werden. Damit die einzelnen Proben bei der späteren Untersuchung im Labor dem richtigen Lieferanten zugeordnet werden können, wird von Hand eine Liste erstellt, in der in der Reihenfolge der angenommenen Volumina die Position eines jeben Probegefäßes in den Stativen zu einer jeden Probe festgehalten wird. In der Praxis kommt es immer wieder vor, daß die ordnungsgemäße Führung der von Hand geführten Liste angezweifelt wird.

Bei einer bekannten Datenerfassungseinrichtung der eingangs genannten Art (DE 34 40 686 A1) beruht die Identifikation der einzelnen Probegefäße und die Zuordnung der bei der Probenahme genommenen Daten für die einzelnen Proben zu den Probegefäßen auf einer am Probegefäß angeordneten Codierung. Mittels eines Lesekopfes wird diese Codierung eines jeden Probegefäßes aufgenommen und derjenigen Position in einem Stativ zugeordnet, an die das jeweilige Probegefäß nach dem Füllvorgang gebracht wird. Die beim Füllen eines jeden Probegefäßes genommenen Daten der Probe werden der Position des Probegefäßes zugeordnet.

Weil einerseits diese Daten einer bestimmten Position des Probegefaßes im Stativ zugeordnet werden und andererseits das Probegefäß dieser Position zugeordnet wird, erlaubt die Codierung an den Flaschen bei einer späteren Auswertung die Identifikatioin der Daten für die einzelne Probe. Voraussetzung für diese Eindeutigkeit ist also, daß jedes Probegefäß eine eigene Codierung hat.

Der Erfindung liegt die Aufgabe zugrunde, eine Datenerfassungseinrichtung zu schaffen, die die Identifizierung der Proben ohne Codes an den Probegefäßen ermöglicht.

Diese Aufgabe wird erfindungsgemäß dadurch gelöst, daß das Stativ aus mehreren nebeneinander oder hintereinander, insbesondere im Kreis angeordneten, jeweils eine Reihe von Probegefäßen aufnehmenden Elementen besteht, die jeweils eine fest angeordnete, maschinenlesbare Markierung tragen und längs denen Codes für die Position der Probegefäße innerhalb einer jeden Reihe vorgesehen sind, und daß der Positionsmelder eine mit dem verfahren Abfüllelement verfahrbare Leseeinrichtung aufweist, mit der die Markierung und die Codes auslesbar sind.

Die Erfindung beruht auf der Erkenntnis, daß für eine eindeutige Identifizierung der genommenen Probe die Codierung der Probegefäße dann überflüssig ist, wenn das Stativ aus einzelnen, jeweils eine Reihe von Probegefäßen aufnehmenden Elementen besteht, diese einzelnen Stativelemente durch an ihnen angebrachte Codes sowohl voneinander als auch hinsichtlich der Reihenfolge der Probegefäße in der Reihe unterschieden werden können. Bei der späteren Auswertung im Labor läßt sich dann aufgrund der Position des einzelnen Probegefäßes im Stativelement das Probegefäß den abgespeicherten Daten eindeutig zuordnen.

Nach einer Ausgestaltung der Erfindung sind die Codes für die Position eines jeden Gefäßes in seiner Reihe auf einem Träger angeordnet, der gegenüber den Stativelementen ortsfest ist. Es ist also nicht erforderlich, daß die Stativelemente selbst Codes für ein jedes Probegefäß aufweisen.

Bei der ersten alternativen Lösung der Erfindung ist das Abfüllelement auf einem an zwei kreuzweise angeordneten Schienen verfahrbaren Kreuzschlitten angeordnet, wobei der Träger für die Codes zusammen mit der einen Schiene des Kreuzschlittens auf dessen anderer Schiene verfahrbar ist. Dabei weist die Leseeinrichtung vorzugsweise zwei Leseköpfe auf, von denen der eine auf der einen Schiene des Kreuzschlittens und der andere auf dessen anderer Schiene gemeinsam mit der einen Schiene verfahrbar sind.

Der andere Lesekopf sollte auf Codes an den Enden der Stativelemente ausgerichtet sein. Es ist möglich, daß Codes nur an den benachbarten Enden der Stativelemente vorgesehen sind. In diesem Fall dürfte es zumindest zweckmäßig sein, wenn das Abfüllen der Probe nur an denjenigen Enden der Stativelemente beginnt, and denen auch die Codes angeordnet sind. Diese Ausgestaltung hat den Vorteil, daß nur ein einziger Lesekopf für die Stativelemente benötigt wird.

Nach einer anderen Ausgestaltung ist vorgesehen, daß der andere Lesekopf aus zwei Teilen besteht, die auf die an gegenüberliegenden Enden der Stativelemente angeordneten Codes ausgerichtet sind. Diese Ausgestaltung ist zwar hinsichtlich des Lesekopfes aufwendiger, bietet aber den Vorteil, daß das Abfüllen der Proben schneller vorsich geht, weil das Abfüllelement bei Erreichen des letzten Probegefäßes in einer Reihe auf das benachbarte Probegefäß der nächsten Reihe überwechseln kann und nicht erst zum Anfang zurückgefahren zu werden braucht.

Im folgenden wird die Erfindung anhand einer Zeichnung näher erläutert. Im einzelnen zeigen:

Fig.1 eine Annahmeanlage für Milch mit einer Probenahmevorrichtung und einer Datenerfassungseinrichtung in schematischer Darstellung,

Fig.2 ein Probeabfüllgerät mit einem Positionsmelder für ein Probenabfüllelement in schematischer Darstellung in Aufsicht,

Fig.3 den Gegenstand gemäß Fig.1 in einer abgewandelten Ausführung und

Fig.4 den Gegenstand gemäß Fig.1 in einer weiteren abgewandelten Ausführung.

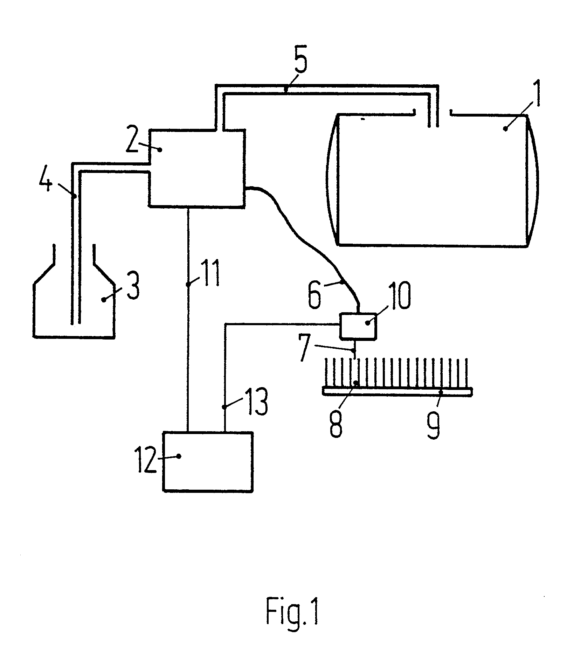

Die in Figur 1 dargestellte Milchannahmeanlage weist einen Sammeltank 1, der auf einem Transportfahrzeug angeordnet ist, sowie eine Förderanlage 2 auf. Die Förderanlage 2 fördert aus einem vom Lieferanten bereitgestellten Gefäß 3 über eine Saugleitung 4 und eine Leitung 5 Milch in den Sammeltank 1. Bei diesem Annahmevorgang wird ein in der Regel mengenproportionaler Teilstrom abgezweigt und daraus eine Probe gebildet, die über eine Leitung 6 und ein Abfüllelement 7, zum Beispiel in Form einer Füllnadel, in einzelne Probegefäße 8 abfüllbar ist, die in einer Reihe in einem Stativelement 9 fest positioniert sind. Mehrere solcher Stativelemente 9 mit Probegefäßen 8 sind nebeneinander in einem nicht dargestellten Stativträger angeordnet. Das Abfüllelement 7 wird von einer im einzelnen noch zu beschreibenden Stellvorrichtung 10 getragen, die die Positionierung des Abfüllelementes 7 in Richtung der X-und Y-Koordinaten erlaubt.

Die Förderanlage 2 weist einen Volumenmesser für die angenommene Milch auf, der über eine Signalleitung 11 Meßwerte für die einzelnen Volumina an eine Datenerfassungseinrichtung 12 liefert. Die Stellvorrichtung 10 weist einen Positionsmelder auf, der über eine Signalleitung 13 die Daten für die Koordinaten des Abfüllelementes 7 bzw. des Probegefäßes 8 an die Datenerfassungseinrichtung 12 liefert. Auf diese Art und Weise ist es möglich, jedem einzelnen Volumen unmittelbar die Position des zugehörigen Probengefäßes 8 zuzuordnen und abzuspeichern.

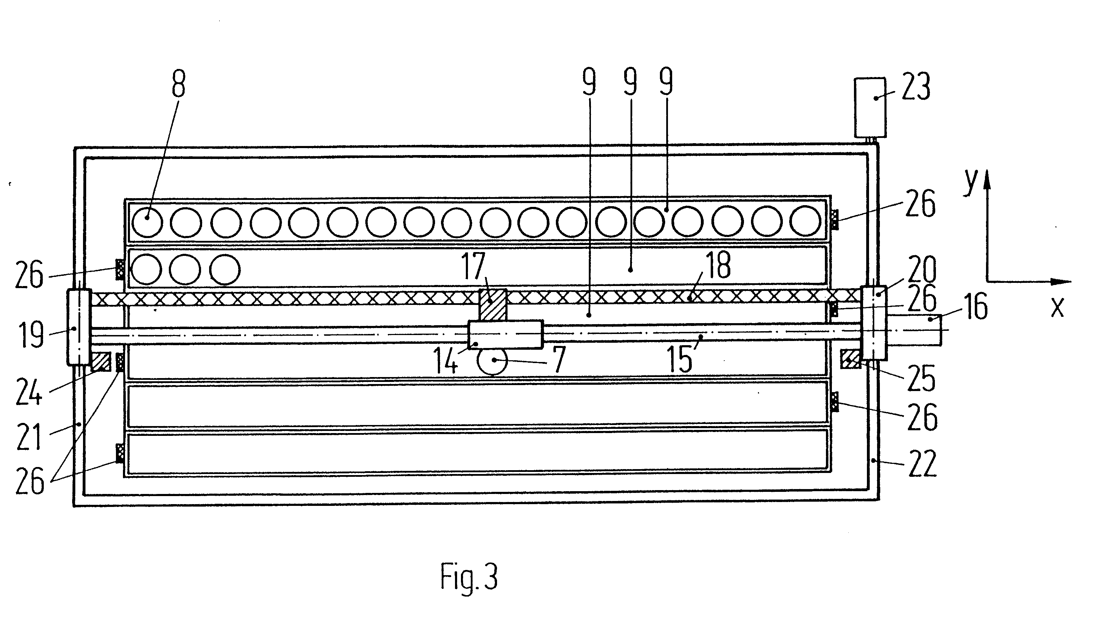

Wie die Figuren 2 und 3 zeigen, sind die jeweils mit einer Reihe von Probegefäßen 8 gefüllten Stativelemente 9 nebeneinander angeordnet, wobei die Probegefäße 8 in Richtung der X- und Y-Koordinaten fest positioniert sind. Das Abfüllelement 7 wird von einem Wagen oder Schlitten 14 getragen, der auf einer in der X-Koordinate angeordneten Schiene 15 verfahrbar ist. Zum Verfahren dient ein Antriebsmotor 16, der beispielsweise über eine nicht dargestellte Antriebsspindel mit dem Wagen 14 gekuppelt ist. Der Wagen 14 trägt ferner einen Lesekopf 17, der auf einem Träger 18 angeordnete Codes für die Position eines jeden Probengefäßes 8 einer jeden Reihe ausleist. Die Schiene 15 und der Träger 18 werden an ihren Enden von Wagen oder Schlitten 19,20 getragen, die auf in der Y-Koordinate verlaufenden Schienen 21,22 angeordnet sind. Diese Wagen 19,20 sind beispielsweise über eine nicht dargestellte Antriebsspindel mit einem Antriebsmotor 23 gekuppelt. Die Wagen 19,20 weisen jeweils einen Lesekopf 24,25 auf, mit denen an den stirnseitigen Enden der Stativelemente 9 codierte Markierungen 26 auslesbar sind. Die Markierungen 26 sind nur an einem Ende eines jeden Stativelementes 9 angeordnet. Während beim Ausführungsbeispiel der Figur 1 die Markierungen 26 an den benachbarten Enden der Stativelemente 9 angeordnet sind, sind sie beim Ausführungsbeispiel der Figur 3 wechselweise an den Enden angeordnet. Bei wechselweise Anordnung gemäß Figur 3 ist es erforderlich, jedem der beiden Wagen 19,20 einen Lesekopf 24,25 zuzuordnen, während beim Ausführungsbeispiel der Figur 1 nur der Lesekopf 24 vorgesehen zu sein braucht.

Die Leseköpfe 24,25 des Positionsmelders übermitteln an die Datenerfassungseinrichtung 12 einen eindeutigen Code für dasjenige Stativelement, in dessen Probegefäße 8 die Proben abgefüllt werden, während der Lesekopf 17 den Code für die jeweilige Position des Probegefäßes 8 innerhalb der Reihe in dem Stativelement 9 liefert. Auf diese Art und Weise wird eindeutig der von einem bestimmten Lieferanten angenommenen Milch die Position der Probe zugeordnet. Bei der späteren Untersuchung im Labor ist an der codierten Markierung sowohl das Stativelement 9 selbst als auch erkennbar, in welcher Richtung die gespeicherten Positionen für die Probegefäße 8 gelten. Verwechslungen durch Fehler bei der Probenerstellung sind damit ausgeschlossen.

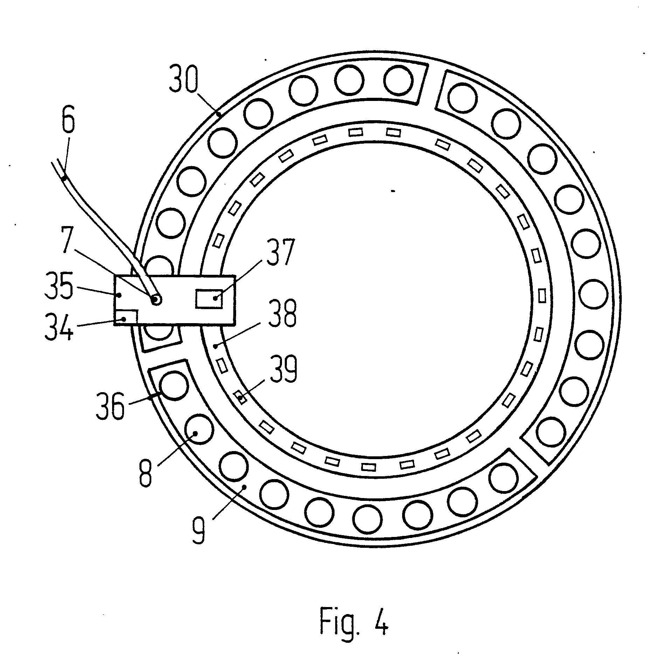

Das Ausführungsbeispiel der Figur 4 unterscheidet sich von den beiden beschriebenen Ausführungsbeispielen im wesentlichen darin, daß die Stativelemente 9 mit der darin in Reihe hintereinander angeordneten Probegefäßen 8 nicht wie bei den anderen Ausführungsbeispielen gerade, sondern kreisbogenförmig ausgebildet und im Kreis angeordnet sind. Die Stativelemente 9 sind auf einem drehbaren Träger 30 angeordnet, so daß sie nacheinander zum Abfüllen einer Probe unter das ortsfeste Abfüllelement 7 gefahren werden können. In kinematischer Umkehr könnte auch das Abfüllelement 7 im Kreis über die einzelnen Probegefäßen gefahren werden. Jedes Stativelement trägt an einem Ende eine auch mit dem Auge erkennbare Marke 36, die von einem Lesekopf 34 erfaßt wird. Innerhalb des Stativkreises ist koaxial ein Codeträger 38 angeordnet, dessen Codes 39 den Probegefäßen 8 zugeordnet sind. Durch die Erfassung des Codes der Markierung 36 eines jeden Stativelementes 9 und der einem jeden Probegefäß 8 zugeordneten Code ist eindeutig die Position eines jeden Probegefäßes 8 bestimmt. Durch die Zuordnung der übrigen Daten für die Milchprobe ist es deshalb möglich, später im Labor die gespeicherten Daten eindeutig dem jeweiligen Probegefäß 8 zuzuordnen. Bei der späteren Untersuchung im Labor läßt sich nämlich anhand der Markierung 36 erkennen, in welcher Reihenfolge die Probegefäße 8 gefüllt worden sind. Über die Daten der Codierung 39 ist dann jedes Probegefäß 8 bestimmbar. Zu Verwechslungen kann es deshalb nicht kommen.

**Ansprüche**

1. Datenerfassungseinrichtung für die Teilvolumina von bei verschiedenen Lieferanten in einem Sammeltank eines Transportfahrzeuges zu sammelnder Milch, mit einem Volumenmesser für die Teilvolumina, einer Probenahmevorrichtung zur Entnahme von Proben aus den Teilvolumina und einem Abfüllgerät für die Proben, das mit seinem Abfüllelement über den in einem Stativ fest positionierten Probegefäßen und relativ zu ihnen verfahrbar angeordnet ist, wobei die Datenerfassungseinrichtung die vom Volumenmesser für die Teilvolumina gelieferten Daten den von einem Positionsmelder gelieferten Daten für die Position des mit der entsprechenden Probe gefüllten Probegefäßes in ihrem Speicher zuordnet,
**dadurch gekennzeichnet,** daß das Stativ aus mehreren nebeneinander angeordneten, jeweils eine Reihe von Probegefäßen (8) aufnehmenden Elementen (9) besteht, die jeweils eine fest angeordnete, maschinenlesbare Markierung (26) tragen und längs denen Codes (18) für die Position der Probegefäße (8) innerhalb einer jeden Reihe vorgesehen sind, und daß der Positionsmelder eine mit dem verfahrbaren Abfüllelement (7) verfahrbare Leseeinrichtung 17,24,25) aufweist, mit der die Markierung (26) und die Codes (18) auslesbar sind.

2. Datenerfassungseinrichtung für die Teilvolumina von bei verschiedenen Lieferanten in einem Sammeltank eines Transportfahrzeuges zu sammelnder Milch, mit einem Volumenmesser für die Teilvolumina, einer Probenahmevorrichtung zur Entnahme von Proben aus den Teilvolumina und einem Abfüllgerät für die Proben, das mit seinem Abfüllelement über den in einem Stativ fest positionierten Probegefäßen und relativ zu ihnen verfahrbar angeordnet ist, wobei die Datenerfassungseinrichtung die vom Volumenmesser für die Teilvolumina gelieferten Daten den von einem Positionsmelder gelieferten Daten für die Position des mit der entsprechenden Probe gefüllten Probegefäßes in ihrem Speicher zuordnet,
**dadurch gekennzeichnet,** daß das Stativ aus mehreren hintereinander, insbesondere im Kreis angeordneten, jeweils eine Reihe von Probegefäßen (8) aufnehmenden Elementen (9) besteht, die jeweils eine fest angeordnete, maschinenlesbare Markierung (36) tragen und längs denen Codes (39) für die Position der Probegefäße (8) innerhalb einer jeden Reihe vorgesehen sind, und daß der Positionsmelder eine mit dem verfahrbaren Abfüllelement (7) verfahrbare Leseeinrichtung (34,37) aufweist, mit der die Markierung (36) und die Codes (39) auslesbar sind.

3. Datenerfassungseinrichtung nach Anspruch 1 oder 2,
**dadurch gekennzeichnet,** daß die Codes (39) für die Position eines jeden Probegefäßes (8) in seiner Reihe auf einem Träger (18,38) angeordnet sind, der in Längsrichtung der Reihen ortsfest ist

4. Datenerfassungseinrichtung nach Anspruch 1 und 3,
**dadurch gekennzeichnet,** daß das Abfüllelement (7) auf einem auf zwei kreuzweise angeordneten Schienen (15,21,22) verfahrbaren Kreuzschlitten angeordnet ist, wobei der Träger (18) für die Codes zusammen mit der einen Schiene (15) des Kreuzschlittens auf dessen anderer Schiene (21,22) vefahrbar ist.

5. Datenerfassungseinrichtung nach einem der Ansprüche 1 und 3 oder 4,
**dadurch gekennzeichnet,** daß die Leseeinrichtung zwei Leseköpfe (17, 24,25) aufweist, von denen der eine auf der einen Schiene (15) des Kreuzschlittens und der andere auf dessen anderer Schiene (21,22) gemeinsam mit der einen Schiene (15) verfahrbar ist.

6. Datenerfassungseinrichtung nach Anspruch 5,
**dadurch gekennzeichnet,** daß der andere Lesekopf (24,25) auf die an den Enden der Stativelemente (9) angeordneten, insbesondere codierten Markierungen (26) ausgerichtet ist.

7. Datenerfassungseinrichtung nach Anspruch 5,

**dadurch gekennzeichnet,** daß der Lesekopf (24,25) aus zwei Teilen besteht, die auf die an gegenüberliegenden Enden der Stativelemente (9) angeordneten, insbesondere codierten Markierungen (26) ausgerichtet sind.

Fig.1

Fig. 2

0 275 072

Fig.3

Fig. 4